# EUROPEAN PATENT APPLICATION

(11) **EP 3 903 769 A1**
(43) Date of publication of application: **03.11.2021**
(21) Application number: 20172409.3
(22) Date of filing: 30.04.2020
(51) Int. Cl.: A61K 9/16, A61K 31/366, A61K 9/14

(54) **SOLID AND LIQUISOLID FORMULATIONS OF CORALLOPYRONIN A**

(71) Applicant: Rheinische Friedrich-Wilhelms-Universität Bonn, 53113 Bonn (DE)
(72) Inventor: WAGNER, Karl G., 53121 Bonn (DE); KROME, Anna Katharina, 53129 Bonn (DE); HÖRAUF, Achim, 53125 Bonn (DE); KÖNIG, Gabriele M., 53127 Bonn (DE); PFARR, Kenneth Michael, 53129 Bonn (DE); SCHIEFER, Andrea, 53639 Königswinter (DE); KEHRAUS, Stefan, 53347 Alfter (DE); HÜBNER, Marc Peter, 53340 Meckenheim (DE)
(74) Representative: Michalski Hüttermann & Partner Patentanwälte mbB

(57) **Abstract**

The present invention relates to a solid or liquisolid formulation comprising corallopyronin A, wherein the formulation comprises an amorphous solid dispersion of corallopyronin A embedded in a water-soluble polymer or corallopyronin A loaded onto porous silica.

## Description

The present invention relates to solid and liquisolid formulations of corallopyronin A.

Corallopyronin A is a natural compound produced by gliding myxobacteria, such as *Corallococcus coralloides.* Corallopyronin A also is denoted methyl N-[(E)-5-[6-hydroxy-5-[(2E,4E,9Z,13E)-8-hydroxy-2,5,9-trimethylpentadeca-2,4,9,13-tetraenoyl]-4-oxopyran-2-yl]hex-1-enyl]carbamate according to the IUPAC nomenclature. Corallopyronin A is known to provide antibacterial properties since 1985. EP 2 520 293 A1 further describes corallopyronin A being a compound usable in the treatment of filariasis.

However, corallopyronin A is a highly lipophilic compound and poorly soluble in water-based solvents. Since solubility is one of the important parameters for bioavailability and oral administration, there is a need of pharmaceutically suitable formulations of corallopyronin A. As the compound corallopyronin A has a semisolid, sticky and oily consistency that is not usable for tableting or filling into capsules or crystallization as a solid compound, and pharmaceutical formulations of corallopyronin A are not available so far.

Therefore, the objective underlying the present invention was to provide a formulation of corallopyronin A that is usable for the manufacture of orally administrable pharmaceutical drug products.

The problem is solved by a solid or liquisolid formulation comprising corallopyronin A, wherein the formulation comprises an amorphous solid dispersion of corallopyronin A embedded in a water-soluble polymer or corallopyronin A loaded onto porous silica.

Surprisingly it was found that embedding corallopyronin A in a water-soluble polymer or loading onto porous silica can provide solid and liquisolid formulations of corallopyronin A that are orally administrable. The solid and liquisolid formulations can be filled into capsules, pressed to tablets, and thus provide usual dosable and convenient oral dosage forms of corallopyronin A.

Solid formulations comprising an amorphous solid dispersion of corallopyronin A embedded in a water-soluble polymer provide advantageously stable formulations. The term "water-soluble" polymers denotes polymers that dissolve, disperse, or swell in water. The water-soluble polymer may be selected from the group comprising cellulose-based polymers, polyvinyl based polymers, poly (butyl methacrylate, 2-dimethyl aminoethyl methacrylate), ethylene oxide/propylene oxide copolymer (Poloxamer), polyvinyl alcohol (PVA), polyvinyl acetate phthalate (PVAP), polyethylene glycol (PEG), alginates, pectins, guar gum, dextrans, chitosan, and starch. Cellulose-based polymers may be selected from the group of hydroxypropylmethylcellulose acetate succinate (HPMCAS), methylcellulose (MC), hydroxyethylcellulose (HEC), hydroxypropyl cellulose (HPC) and hydroxyethylmethylcellulose (HEMC). Polyvinyl based polymers may be selected from the group of polyvinylpyrrolidone (PVP), polyvinylpyrrolidone/vinylacetate copolymer (PVP/VA), and polyvinylcaprolactam/polyvinyl acetate/polyethylene glycol copolymer.

In preferred embodiments, the water-soluble polymer is selected from the group of polyvinylpyrrolidone (PVP), polyvinylpyrrolidone/vinylacetate copolymer (PVP/VA), hydroxypropyl cellulose (HPC), hydroxypropyl methylcellulose (HPMC) and polyethylene glycol (PEG), preferably selected from PEG 6000, PEG 12000, and PEG 20000.

In embodiments, the water-soluble polymer comprises corallopyronin A in an amount in a range of ≥ 10 weight% to ≤ 50 weight%, preferably in an amount in a range of ≥ 15 weight% to ≤ 40 weight%, further preferred in an amount in a range of ≥ 20 weight% to ≤ 30 weight%, based on a weight of 100 weight% of the water-soluble polymer and corallopyronin A.

In preferred embodiments, the formulation comprises corallopyronin A embedded in polyvinylpyrrolidone. Particularly amorphous solid dispersions of corallopyronin A embedded in polyvinylpyrrolidone showed advantageous solubility and stability.

In another embodiment, corallopyronin A dispersed in a solvent and is adsorbed onto porous silica to form a liquisolid formulation. The solvent may be selected from non-volatile solvents, preferably from propylene carbonate, PEG 200-400, or medium-chain triglycerides such as miglyol® 812. The term "medium-chain triglycerides" refers to fatty acids that have a chain length of 6-12 carbon atoms. The solvent preferably is propylene carbonate.

The term "liquisolid system" refers to formulations formed by conversion of liquid drugs, drug suspensions, or drug solutions in non-volatile solvents, into dry, non-adherent, free-flowing or particulate and compressible powder mixtures by blending the suspension or solution with selected carriers. The concept of liquisolid technique relies on that when a drug dissolved in the liquid vehicle is incorporated into a carrier material which has a porous surface, loading such as adsorption of the liquid onto the internal and external surfaces of the porous carrier particles occur. The carrier material having high adsorptive properties and large specific surface area gives the liquisolid system the desirable flow characteristics. The porous silica may be selected from mesoporous, microporous silica, and macroporous silica.

Preferably, the porous silica is a mesoporous silica. Advantageously, corallopyronin A may be adsorbed onto porous silica. Propylene carbonate as a solvent provides low toxicity and low viscosity. Corallopyronin A may be adsorbed onto the porous silica in an amount in a range of ≥ 10 weight% to ≤ 70 weight%, preferably in an amount in a range of ≥ 20 weight% to ≤ 50 weight%, based on a weight of 100 weight% of the liquisolid formulation comprising the porous silica, corallopyronin A and propylene carbonate.

Embedding corallopyronin A in a water-soluble polymer or loading onto porous silica can provide orally administrable solid and liquisolid formulations of corallopyronin A. In preferred embodiments, the formulation is a solid or liquisolid oral formulation. In embodiments, the solid or liquisolid formulation is formulated into a capsule, a tablet, granules, pills, pellets, or micro-tablets.

A further aspect of the present invention relates to a pharmaceutical solid dosage form, comprising the solid or liquisolid formulation comprising corallopyronin A according to the invention. In embodiments, the solid dosage form is selected from a capsule, a tablet, granules, pills, pellets or micro-tablets. For the further description of the solid or liquisolid pharmaceutical formulation comprising corallopyronin A, reference is made to the description above.

A further aspect of the present invention relates to a process of manufacturing a solid or liquisolid formulation according to the invention, comprising the steps of:
a) dispersing, dissolving or otherwise introducing corallopyronin A into a solvent to form a liquid mixture,
b) selecting a substrate from porous silica or a water-soluble polymer,
c) admixing the liquid mixture of step a) and the substrate of step b), and
d) optionally removing excess solvent from the mixture obtained in step c) to form a solid or liquisolid formulation.

The solid and liquisolid formulation formulations can be manufactured with little effort and standard equipment and thus are useful for preclinical and clinical development of pharmaceutical ingredients. Also industrial processing is possible in energy and cost effective, particularly compared to the manufacture of amorphous solid dispersions.

The solvent may be an organic solvent selected from ethanol, methanol, isopropanol, dichloromethane, acetone, tert-butanol, propylene carbonate, a C6 to C12 triglyceride, preferably a C8 or C10 triglyceride, a polymer which is liquid at ambient temperature such as a polyethylene glycol (PEG), preferably selected from PEG 400, PEG 300 and PEG 200, or mixtures thereof. The term "C6 to C12" triglycerides" refers to fatty acids that have a chain length of 6-12 carbon atoms.

The solid or liquisolid formulation may be orally administered. It is however preferred to further formulate the solid or liquisolid formulation into a usual pharmaceutical solid dosis form, such as a capsule, granules or a tablet. In embodiments of the process, in a further step e) the solid or liquisolid formulation is formed into a tablet, granules, pills, pellets or micro-tablets, preferably into capsules, granules or tablets. In embodiments, the process thus is a process for manufacturing capsules, granules or tablets comprising the solid or liquisolid formulation.

For manufacturing capsules, tablets or granules, the solid or liquisolid formulation may be filled into capsules, mixed with tableting additives and compressed to tablets, or wet or dry granulation can be applied to form granules, respectively. These methods are standard procedures known to a person skilled in the art and for example may be performed using usual tableting machines, capsule filling machines or suitable devices for wet granulation, e.g. high shear mixers, fluidized air granulators, or dry granulation, e.g. roller compactors. Preferably, the solid or liquisolid formulation is filled into capsules or mixed with tableting additives and compressed to tablets.

In a preferred embodiment, a solid formulation comprising corallopyronin A embedded in a water-soluble polymer is manufactured, the process comprising the steps of:
a) dispersing, dissolving or otherwise introducing corallopyronin A in an organic solvent to form a liquid mixture,
b) selecting a water-soluble polymer,
c) admixing the liquid mixture of step a) and the water-soluble polymer of step b), and
d) removing excess solvent from the mixture obtained in step c) to form a solid formulation.

In preferred embodiments for manufacturing a solid formulation comprising corallopyronin A embedded in a water-soluble polymer, the solvent is an organic solvent selected from ethanol, methanol, isopropanol, dichloromethane, acetone or tert-butanol. A preferred organic solvent is ethanol. Ethanol is preferred due to its low toxic potential. Advantageously, high amounts of corallopyronin A can be dissolved in ethanol, such as 100 mg/mL.

The dispersing, dissolving or otherwise introducing in step a) may be performed at a temperature in a range from ≥ 10 °C to ≤ 50 °C, preferably in a range from ≥ 15 °C to ≤ 25 °C, and/or may be performed using ultrasonic dissolving, vortexing, or mixing using magnetic mixers or blade agitators.

The water-soluble polymer may be selected from the group comprising cellulose-based polymers, polyvinyl based polymers, poly (butyl methacrylate, 2-dimethyl aminoethyl methacrylate), ethylene oxide/propylene oxide copolymer (Poloxamer), polyvinyl alcohol (PVA), polyvinyl acetate phthalate (PVAP), polyethylene glycol (PEG), alginates, pectins, guar gum, dextrans, chitosan, and starch. Cellulose-based polymers may be selected from the group of hydroxypropylmethylcellulose acetate succinate (HPMCAS), methylcellulose (MC), hydroxyethylcellulose (HEC), hydroxypropyl cellulose (HPC) and hydroxyethylmethylcellulose (HEMC). Polyvinyl based polymers may be selected from the group of polyvinylpyrrolidone (PVP), polyvinylpyrrolidone/vinylacetate copolymer (PVP/VA), and polyvinylcaprolactam/polyvinyl acetate/polyethylene glycol copolymer. The water-soluble polymer preferably is selected from the group of polyvinylpyrrolidone (PVP), polyvinylpyrrolidone/vinylacetate copolymer (PVP/VA), hydroxypropyl cellulose (HPC), hydroxypropyl methylcellulose (HPMC) and polyethylene glycol (PEG), preferably selected from PEG 6000, PEG 12000 or PEG 20000.

A water-soluble polymer may be selected in step b) and in step c) may be dispersed, dissolved or otherwise introduced into the liquid mixture of corallopyronin A in the organic solvent of step a). In other embodiments, in step b) the water-soluble polymer may be dispersed or dissolved in water, an organic solvent, or a mixture thereof. In these embodiments, in step c) liquid mixtures of step a) and of step b) may be mixed and may result in a mixture of organic solvents or organic solvent and water.

In step d) excess solvent is removed from the mixture to form a solid oral formulation. The solvent may for example be removed by evaporating, such as by drying the formulation at ambient temperature or by using vacuum. The solvent or solvent mixture also may be removed by spray drying, freeze drying or spray freeze drying the mixture of step c) whereby also a solid oral formulation comprising corallopyronin A embedded in a water-soluble polymer is obtained. The evaporating or spray drying may be performed using standard equipment such as usual rotary evaporators and spray dryers.

In other embodiments of the process, a solid or liquisolid formulation comprising corallopyronin A adsorbed to mesoporous silica is produced. In embodiments, a solid or liquisolid formulation comprising corallopyronin A adsorbed to mesoporous silica may be manufactured, the process comprising the steps of:
a) dispersing, dissolving or otherwise introducing corallopyronin A into a solvent to form a liquid mixture,
b) selecting a porous silica,
c) admixing the liquid mixture of step a) and the porous silica of step b), and
d) optionally removing excess solvent from the mixture obtained in step c) to form a solid or liquisolid formulation.

The porous silica may be selected from mesoporous, microporous silica, and macroporous silica. Preferably, the porous silica is mesoporous silica. Mesoporous silica advantageously allows for corallopyronin A being adsorbed onto the silica surface.

A solid formulation comprising corallopyronin A adsorbed to mesoporous silica may be manufactured, the process comprising the following steps of:
a) dispersing, dissolving or otherwise introducing corallopyronin A in an organic solvent,
b) selecting a mesoporous silica,
c) admixing the liquid mixture of step a) and the mesoporous silica of step b), and
d) removing excess solvent from the mixture obtained in step c) to form a solid formulation.

The organic solvent may be selected from ethanol, methanol, isopropanol, dichloromethane, acetone, and tert-butanol. Preferably, the organic solvent is ethanol.

Preferably, in step d) excess solvent is removed by drying the mixture at a temperature in a range from ≥ 15 °C to ≤ 60 °C, preferably at a temperature in a range from ≥ 20 °C to ≤ 40 °C, thereby producing a solid oral formulation comprising corallopyronin A adsorbed to mesoporous silica.

A liquisolid formulation comprising corallopyronin A adsorbed to mesoporous silica may be manufactured, the process comprising the following steps of:
a) dispersing, dissolving or otherwise introducing corallopyronin A into propylene carbonate or a mixture of propylene carbonate and a further solvent to form a liquid mixture,
b) selecting a mesoporous silica,
c) admixing the liquid mixture of step a) and the mesoporous silica of step b), and
d) optionally removing excess propylene carbonate or excess mixture of propylene carbonate and the further solvent from the mixture obtained in step c) to form a liquisolid formulation.

Preferably, in step a) corallopyronin A is dissolved in propylene carbonate or a mixture of propylene carbonate and a further solvent, thereby producing a liquisolid oral formulation comprising corallopyronin A in propylene carbonate or a mixture of propylene carbonate and a further solvent adsorbed to mesoporous silica. The further solvent may be selected from dimethyl sulfoxide (DMSO), ethanol, methanol, isopropanol, dichloromethane, acetone, tert-butanol, or a polymer which is liquid at ambient temperature (20±2°C) such as a polyethylene glycol (PEG) preferably selected from PEG 400, PEG 300 and PEG 200. Preferred further solvents are dimethyl sulfoxide (DMSO) and ethanol.

Optionally, excess propylene carbonate or a mixture of propylene carbonate and a further solvent from the mixture obtained in step c) may be removed. It is however, preferred to add only as much propylene carbonate or a mixture of propylene carbonate to the mesoporous silica, which can be adsorbed. Excess propylene carbonate or solvent mixture may for example be removed by evaporating, such as by using vacuum drying.

A further aspect of the present invention relates to a solid or liquisolid formulation comprising corallopyronin A or a pharmaceutical solid dosage form obtained by the process according to the invention. For the description of the solid or liquisolid formulation and the pharmaceutical solid dosage forms, reference is made to the description above. Preferred pharmaceutical solid dosage forms are selected from a capsule, a tablet, granules, pills, pellets or micro-tablets.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: The biphasic dissolution profile of the organic phase of the amorphous formulation of corallopyronin A (CorA) embedded in polyvinylpyrrolidone (PVP) compared to pure corallopyronin A.
- Figure 2: The biphasic dissolution profile of corallopyronin A embedded in polyvinylpyrrolidone.
- Figure 3: The plasma concentration time profile of the liquid formulation and the solid formulation of corallopyronin A (CorA) embedded in polyvinylpyrrolidone *in vivo.*
- Figure 4: The biphasic dissolution profile of an amorphous formulation of corallopyronin A in polyvinylpyrrolidone/ vinylacetate copolymer.
- Figure 5: The biphasic dissolution profiles of an amorphous formulation of corallopyronin A embedded in polyethylene glycol.
- Figure 6: The biphasic dissolution profiles of an amorphous formulation of corallopyronin A embedded in hydroxypropyl cellulose.
- Figure 7: The biphasic dissolution profiles of the neat corallopyronin A (CorA) compound.

### Example 1: Manufacture of an amorphous corallopyronin A formulation by polymer film casting using polyethylene glycol

0.1501 g Corallopyronin A (produced by the Myxobacterium *Corallococcus coralloides* B035; Helmholtz Centre for Infection Research, Dept. Microbial Drugs, Braunschweig) was weighted in a beaker and 2 ml ethanol were added. Thereafter the beaker was covered with parafilm and placed on an ultrasonic bath (Bandelin, Sonorex Digitec). The temperature of the bath was held to < 25 °C by replacing the water every 10 min. After 20 min the corallopyronin A was fully dissolved. In a further beaker 0.614 g polyethylene glycol (PEG 20000, Roth) was weighted and 10.825 mL ethanol and 0.675 mL water were added to the beaker. The beaker was covered with parafilm and placed on the ultrasonic bath for 60 min. The temperature of the bath was held to < 25 °C by replacing the water every 10 min. After 60 min the polymer was fully dissolved. Both solutions were mixed together with a metal spatula and placed on the ultrasonic bath for another 5 min. After mixing the mixture was transferred into a 250 mL perfluoroxy alkane (PFA) round-bottom flask and dried at 25 °C for 24 hours at a vacuum of 600 - 0 mbar and 50 rpm on a rotary evaporator (Laborata 4000 efficient, Heidolph). Solvent evaporation yielded an amorphous solid formulation of corallopyronin A embedded in polyethylene glycol. The drug load was calculated to about 20 weight%.

### Example 2: Manufacture of an amorphous corallopyronin A formulation by polymer film casting using hydroxypropyl cellulose

0.1539 g Corallopyronin A (produced by the Myxobacterium *Corallococcus coralloides* B035; Helmholtz Centre for Infection Research, Dept. Microbial Drugs, Braunschweig) was weighted in a beaker and 2 mL ethanol were added. Thereafter, the beaker was covered with parafilm and placed on an ultrasonic bath (Bandelin, Sonorex Digitec). The temperature of the bath was held to ≤ 25 °C by replacing the water every 10 min. After 20 min the corallopyronin A was fully dissolved. In a further beaker 0.6001 g hydroxypropyl cellulose super super fine (HPC SSL, Nisso) was weighted and 11.5 mL ethanol was added to the beaker. The beaker was covered with parafilm and placed on the ultrasonic bath. The temperature of the bath was held to ≤ 25 °C by replacing the water every 10 min. After 40 min the polymer was fully dissolved. Both solutions were mixed together with a metal spatula and placed on the ultrasonic bath for another 5 min. After mixing the mixture was transferred into a 250 mL perfluoroxy alkane (PFA) round-bottom flask and dried at 25 °C for 24 hours at a vacuum of 600 - 0 mbar and 50 rpm on a rotary evaporator (Laborata 4000 efficient, Heidolph). Solvent evaporation yielded an amorphous solid formulation of corallopyronin A embedded in hydroxypropyl cellulose. The drug load was calculated to 20 weight%.

### Example 3: Manufacture of an amorphous formulation of corallopyronin A embedded in polyvinylpyrrolidone by spray drying

1.6022 g Corallopyronin A (produced by the Myxobacterium *Corallococcus coralloides* B035; Helmholtz Centre for Infection Research, Dept. Microbial Drugs, Braunschweig) was weighted in a beaker and 16.0 mL ethanol was added. Thereafter, the beaker was covered with parafilm and placed on an ultrasonic bath (Bandelin, Sonorex Digitec). The temperature of the bath was held to < 25 °C by replacing the water every 10 min. After 40 min the corallopyronin A was fully dissolved. In a further beaker 6.4237 g polyvinylpyrrolidone (PVP-K30 LP, BASF) was weighted in and 51.0 mL ethanol was added. The beaker was covered with parafilm and placed on the ultrasonic bath at 25 °C. After 5 min the polymer was dissolved. Both solutions were mixed together with a metal spatula and placed on the ultrasonic bath for another 5 min. After mixing, the pipe of the spray dryer (B-290; BUCHI) was connected to the solution and the spray drying process started with an inlet temperature of 85 °C, an outlet temperature of 59 °C, aspirator 100% and pump running at 20%. Nitrogen was used as drying gas and the flow rate was set to 5.6 mL/min. Spray drying yielded an amorphous solid formulation of corallopyronin A embedded in polyvinylpyrrolidone. The drug load was calculated to about 20 weight%.

### Example 4: Manufacture of an amorphous formulation of corallopyronin A embedded in polyvinylpyrrolidone/vinylacetate copolymer by spray drying

1.6008 g Corallopyronin A (produced by the Myxobacterium *Corallococcus coralloides* B035; Helmholtz Centre for Infection Research, Dept. Microbial Drugs, Braunschweig) was weighted in a beaker and 16 mL ethanol were added. Thereafter, the beaker was covered with parafilm and placed on an ultrasonic bath (Bandelin, Sonorex Digitec). The temperature of the bath was held to ≤ 25 °C by replacing the water every 10 min. After 40 min the corallopyronin A was fully dissolved. In a further beaker 6.4032 g polyvinylpyrrolidone/vinylacetate copolymer (Kollidon VA 64; BASF) was weighted and 51.0 mL ethanol was added. The beaker was covered with parafilm and placed on the ultrasonic bath at 25 °C. After 5 min the polymer was dissolved. Both solutions were mixed together with a metal spatula and placed on the ultrasonic bath for another 5 min. After mixing the pipe of the spray dryer (B-290; BUCHI) was connected to the solution and the spray drying process started with an inlet temperature of 85 °C, an outlet temperature of 59 °C, aspirator 100% and pump running at 20%. Nitrogen was used as drying gas and the flow rate was set to 4.5 mL/min. Spray drying yielded an amorphous solid formulation of corallopyronin A embedded in polyvinylpyrrolidone/vinylacetate copolymer. The drug load was calculated to about 20 weight%.

### Example 5: Manufacture of a corallopyronin A formulation by adsorption to mesoporous silica

0.1012 g Corallopyronin A (produced by the Myxobacterium *Corallococcus coralloides* B035; Helmholtz Centre for Infection Research, Dept. Microbial Drugs, Braunschweig) was weighted in a 3 mL glass vial and 300 µl ethanol was added. Thereafter, the glass vial was covered with parafilm and placed on an ultrasonic bath (Bandelin, Sonorex Digitec). The temperature of the bath was held to ≤ 25 °C by replacing the water every 10 min. After 20 min the corallopyronin A was fully dissolved. Thereafter, 0.1021 g of mesoporous silica (Syloid XRD 3050, Grace GmbH, Worms) was added to the glass vial and the mixture was mixed with a metal spatula and dried for 24 h under the fume hood at 20 °C. Drying yielded a solid formulation of corallopyronin A loaded onto mesoporous silica. The drug load was calculated to about 50 weight%.

### Example 6: Pharmacological testing of the amorphous formulation of corallopyronin A embedded in polyvinylpyrrolidone

The amorphous formulation of corallopyronin A embedded in polyvinylpyrrolidone by spray drying as prepared in example 3 was tested for dissolution performance, pharmacokinetics and stability.

### 6.1 Biphasic dissolution tests

Biphasic release is a process in which, in addition to solubility in an aqueous medium, adsorption into the intestinal wall is demonstrated using an organic phase. The transition to the organic phase can be understood as an absorption of active substances into the blood. The biphasic release experiments were investigated using a fully automated biphasic dissolution apparatus (BiPHa+) as described in A. Denninger et al., Pharmaceutics 2020, 12, 237.

To simulate the gastrointestinal properties to obtain *in vivo* predictive results, the pH-profile, bile salt concentration and gastrointestinal passage time were adjusted in the aqueous phase to mimic human gut conditions. Additionally, an organic phase above the aqueous phase imitates the fraction absorbed from the gut. An organic phase of 1-decanol above the aqueous phase imitated the fraction absorbed from the gut. As a test model, the profile of a person without prior food intake was chosen. Therefore, FaSSIF-V2 medium, which represents the bile salts concentration of a fasted human was used. FaSSIF-V2 medium is a mixture of a phosphate and a citrate buffer system, which facilitates comparable *in vivo* buffer capacities. To generate an in-situ biorelevant aqueous medium for the fasted state, biorelevant surfactants, namely sodium-taurocholate (3 mM) and lecithin (0.2 mM), were added.

Prior to the start of the experiments, both phases, 1-decanol and the acidic aqueous phase, were saturated with each other. First, about 10 mg of the API corallopyronin A in the respective formulation was added to 50 mL HCl (0.1 M), simulating the stomach. During the first thirty minutes the formulation disintegrated / dispersed in 50 mL of 0.1N HCl.

After 30 min FaSSIF-V2 concentrate (sodium-taurocholate and lecithin) was added simultaneously to the addition of citrate-phosphate buffer (tri-potassium phosphate and potassium citrate) resulting in a first pH-shift from pH 1 to pH 5.5, simulating the duodenum. Thereafter, 50 mL decanol was added. The last pH-shift from 5.5 to 6.8 after 90 minutes was gradually adjusted by adding more citrate-phosphate buffer, representing the jejunum and ileum (final concentrations: 3 mM sodium-taurocholate, 0.2 mM lecithin, 525 mM tri-potassium phosphate and 225 M potassium citrate). The adjusted buffers were titrated by 0.1 M NaOH and 0.1 M HCl (pH 5.5-6.8). The whole dissolution took 4.5 hours.

The concentration profiles of the aqueous and organic phase were measured online continuously with an Agilent 8454 UV-Vis spectrometer (Waldbronn, Germany) and quantified on the compound-specific wavelength. Three independent dissolution tests were performed.

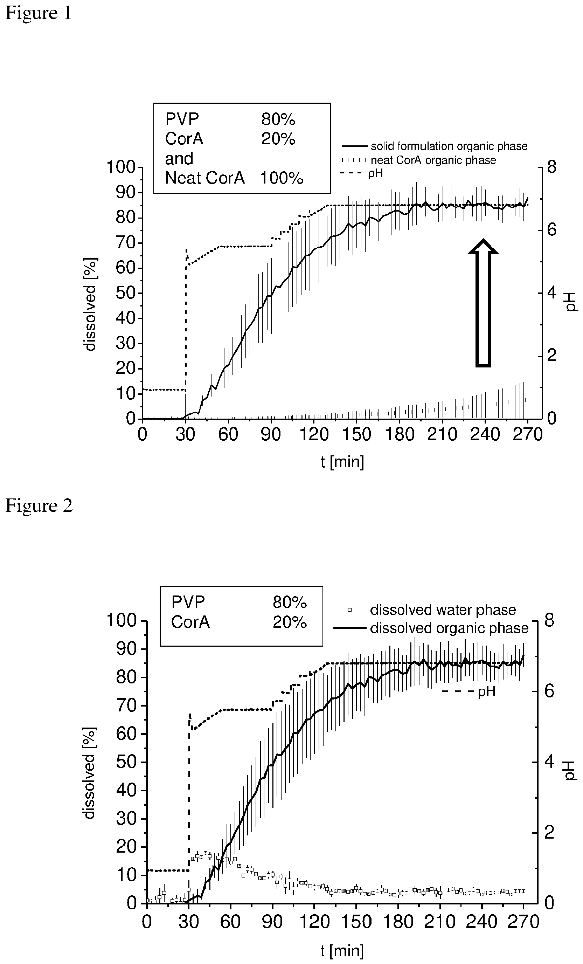

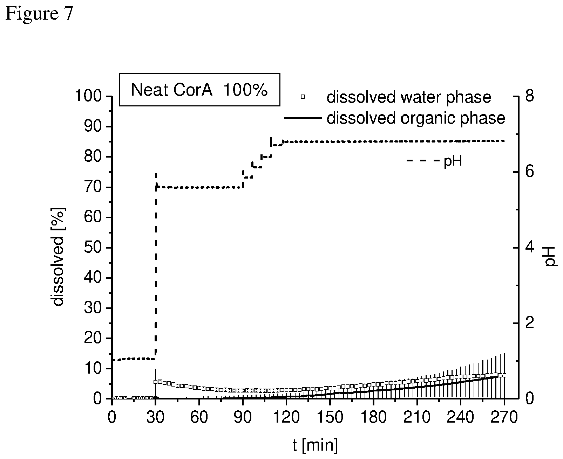

Figure 1 shows the pH profile and illustrates the biphasic dissolution profile of the amorphous formulation of corallopyronin A embedded in polyvinylpyrrolidone as prepared in example 3 compared to neat corallopyronin A. As can be taken from figure 1, the amorphous formulation of corallopyronin A provided 87.6 % predicted absorption. The formulation thus provides very good *in vitro* performance. Compared to the solubility of the neat compound corallopyronin A, as described in comparative example 10, the biphasic dissolution model for human absorption shows a more than 10 fold increase of fraction absorbed due to solubility enhanced amorphous solid formulation. Individual dissolution graphs of corallopyronin A embedded in polyvinylpyrrolidone and of the pure corallopyronin A compound are provided in figures 2 and 7, respectively.

### 6.2 Pharmacokinetic analysis

Pharmacokinetic analysis was performed *in vivo* in fed BALB/c female mice (n=4) (Study DZIF11). The solid formulation was suspended right before administration in PBS (phosphate buffered saline; pH=7.4) and was administered via gavage (36 mg/kg CorA, 10 mL/kg). Blood samples from the vena facialis were collected after 5, 10, 15, 30, 180, and 480 min. The samples were centrifuges for 10 min at 4 °C and 15000 g. The generated plasma was mixed in a ration of 1:3 with ice-cold acetonitrile (Bernd Kraft GmbH, Duisburg). The mixture was vortexed for 10 sec and centrifuged for 25 min at 4 °C and 11600 g. The HPLC measurements were performed on a Waters Alliance e2695 separation module connected to a waters 2998 PDA detector. A waters XBridge® Shield RP₁₈, 3.5 µm, 2.1x100 mm, 130 A column was used. For the gradient method starting from 70% A/ 30% B to 20% A/ 80 % B stepwise within 30 min, the mobile phases A (acetonitrile/water (Bernd Kraft GmbH, Duisburg) 5/95 with 5 mM ammonium acetate (Merck KGaA, Darmstadt) and 40 µL acetic acid (Sigma-Aldrich Chemie GmbH, Steinheim) per Liter) and B (acetonitrile/water 95/5 with 5 mM ammonium acetate and 40 µL acetic acid per Liter) enabled to quantitate corallopyronin A precisely at 300 nm at a flow rate of 0.3 mL/min and a column temperature of 30 °C. The samples were quantitated via external reference standard. The content of the corallopyronin A reference material was analyzed by ¹H-NMR.
Plasma concentration-time profiles were generated and pharmacokinetic parameters calculated.

To calculate the absolute bioavailability of the oral formulation, an intravenous (IV) profile was conducted. Therefore, corallopyrinine A (CorA) was prepared in a liquid formulation (propylene glycol (20%) (Caesar & Lorenz GmbH, Hilden); Kolliphor® HS-15 (20 %) (Sigma-Aldrich Chemie GmbH, Taufkirchen); PBS pH 7.4 (60 %)). The prepared solution was then administered intravenously (36 mg/kg CorA, 5 mL/kg) in the tail vein. The following table 1 shows the results of the pharmacokinetic analysis.

**Table 1: Results of the pharmacokinetic analysis**

| pharmacokinetic analysis (median values) | IV | PO |
|---|---|---|
| | liquid CorA formulation (PG; K-HS15; PBS) | solid CorA formulation (PVP) |
| | (36 mg/kg) | (36 mg/kg) |
| AUC ₍₀₋₈ₕ₎ [µg*h/mL] | 115.5 | 67.8 |
| AUC _{(0-inf)} [µg*h/mL] | 127.7 | 75.9 |
| C ₘₐₓ [µg/mL] | 119.6 | 64.3 |
| t ₘₐₓ [min] | 5* *first measured value | 10 |
| F_{abs} [%] | 100 | 59 |

Table 1 shows the pharmacokinetic parameters after the IV administration of the liquid formulation and the PO administration of the solid formulation. The area under the concentration-time over all time was calculated for the IV application as 127.7 µg*h/mL (median value) and for the PO application as 75.9 µg*h/mL (median value). Based on the AUC results, the absolute bioavailability after the PO was calculated to be 59% (F_{abs}=(AUC_{(0-inf)} IV/ AUC_{(0-inf)} PO)*100), demonstrating a high oral bioavailability in mice due to the enhanced solubility formulation principle.

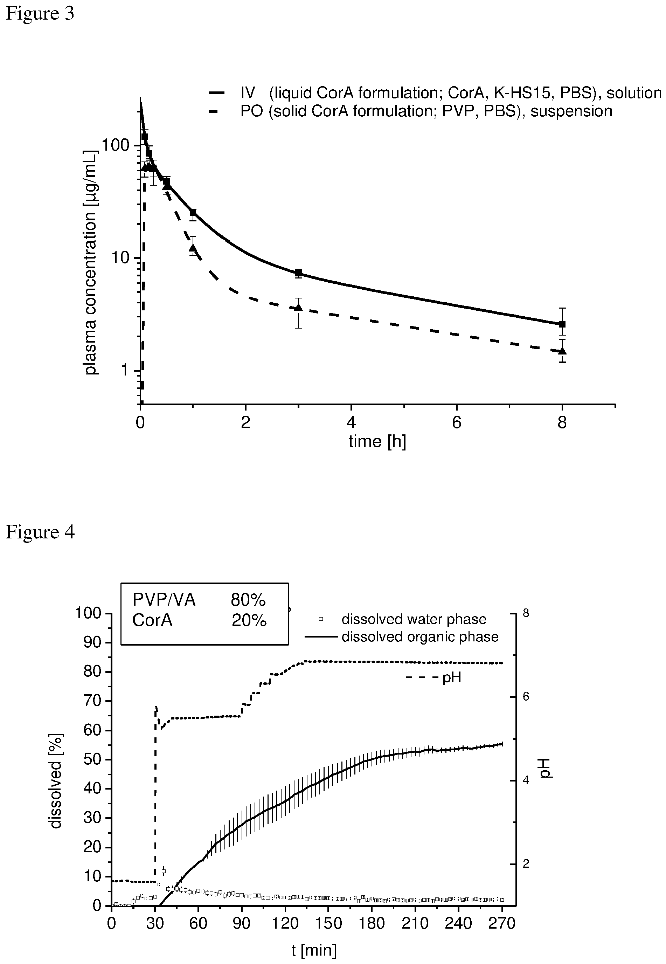

Figure 3 illustrates the plasma concentration-time profile of the IV administration of the CorA solution and the PO administration of the solid formulation *in vivo.* The fast course and high measured values of the PO curve demonstrate the solubility enhancement of the formulation principle since a fast and high oral absorption of an active pharmaceutical ingredient is only possible when the API is dissolved and transported via passive or active transport into the blood circulation. Due to the lipophilic character of corallopyronin A a sufficient absorption through membranes was predicted in combination with the solubility enhanced formulation principle a sufficient oral bioavailability in mice was demonstrated.

### 6.3 Stability analysis

The stability of the spray dried amorphous formulation of corallopyronin A embedded in polyvinylpyrrolidone was analyzed after storing samples in drying cabinets at 25 °C / 60% relative humidity (RH) and under accelerated conditions at 40 °C/75% RH for 7, 14, 28 days and 3 months. The samples were stored in closed twist-off glass vials under nitrogen and in the presence of silica desiccant. The content was analyzed by High-Performance Liquid Chromatography with Diode-Array Detection (HPLC-DAD). The samples were, therefore, dissolved in acetonitrile (Bernd Kraft GmbH, Duisburg). The HPLC measurements were performed on a Waters Alliance e2695 separation module connected to a waters 2998 PDA detector. A waters XBridge® Shield RP₁₈, 3.5 µm, 2.1x100 mm, 130 A column was used. For the gradient method starting from 70% A/ 30% B to 20% A/ 80 % B stepwise within 30 min, the mobile phases A (acetonitrile/water (Bernd Kraft GmbH, Duisburg) 5/95 with 5 mM ammonium acetate (Merck KGaA, Darmstadt) and 40 µL acetic acid (Sigma-Aldrich Chemie GmbH, Steinheim) per Liter) and B (acetonitrile/water 95/5 with 5 mM ammonium acetate and 40 µL acetic acid per liter) enabled to quantitate corallopyronin A precisely at 300 nm at a flow rate of 0.3 mL/min and a column temperature of 30 °C. The samples were quantitated via external reference standard. The content of the corallopyronin A reference material was analyzed by ¹H-NMR. The following table 2 summarizes the results of the stability analysis.

**Table 2: Results of the stability analysis**

| Storage Duration | Storage Sondition | CorA Content [%] |
|---|---|---|
| 0 | | 100 |
| 7 days | 25 °C; 60% RH | 100 |
| 14 days | 25 °C; 60% RH | 100 |
| 28 days | 25 °C; 60% RH | 98 |
| 3 months | 25 °C; 60% RH | 98 |
| 7 days | 40 °C; 75% RH | 98 |
| 14 days | 40°C; 75% RH | 97 |
| 28 days | 40 °C; 75% RH | 93 |
| 3 months | 40 °C; 75% RH | 83 |

The results of table 2 show that the spray dried amorphous formulation of corallopyronin A embedded in polyvinylpyrrolidone showed good long term stability at 25 °C, and also provided sufficient stability under conditions of accelerated decay. Compared to the highly instable compound corallopyronin A as shown in example 10.2, the spray dried formulation with polyvinylpyrrolidone provides a considerable stability enhancement. This shows that formulations embedding corallopyronin A in water-soluble polymers such as polyvinylpyrrolidone provides solubility and stability enhancements.

### Example 7: Pharmacological testing of the amorphous formulation of corallopyronin A embedded in polyvinylpyrrolidone/vinylacetate copolymer

### 7.1 Biphasic dissolution tests

The amorphous formulation of corallopyronin A embedded in polyvinylpyrrolidone/ vinylacetate copolymer by spray drying as prepared in example 4 was tested for dissolution performance as described in example 6.1.

The figure 4 shows the pH profile and illustrates the biphasic dissolution profile of the amorphous formulation of corallopyronin A in polyvinylpyrrolidone/ vinylacetate copolymer. As can be taken from figure 4, the formulation provided 55.4 % predicted absorption. The formulation thus provides good *in vitro* performance.

### 7.2 Stability analysis

The stability of the amorphous formulation of corallopyronin A in polyvinylpyrrolidone/ vinylacetate copolymer was tested as described in example 6.3. The following table 3 summarizes the results of the stability analysis.

**Table 3: Results of the stability analysis**

| Storage Duration | Storage Sondition | CorA Content [%] |
|---|---|---|
| 0 | | 100 |
| 7 days | 25 °C; 60% RH | 100 |
| 14 days | 25 °C; 60% RH | 100 |
| 28 days | 25 °C; 60% RH | 99 |
| 3 months | 40 °C; 75% RH | 98 |
| 7 days | 40°C; 75% RH | 100 |
| 14 days | 40 °C; 75% RH | 99 |
| 28 days | 40 °C; 75% RH | 98 |
| 3 months | 25 °C; 60% RH | 88 |

The results of table 3 show that the spray dried amorphous formulation of corallopyronin A embedded in polyvinylpyrrolidone/ vinylacetate copolymer (PVP/VA) showed good long term stability at 25 °C, and also provided sufficient stability under conditions of accelerated decay. This shows that also a stability enhancement was achieved by embedding the corallopyronin A in PVP/VA. This shows that also formulations embedding corallopyronin A in PVP/VA provide solubility and stability enhancements.

### Example 8: Biphasic dissolution test of the amorphous formulation of corallopyronin A embedded in polyethylene glycol

The amorphous formulation of corallopyronin A embedded in polyethylene glycol by film casting as prepared in example 1 was tested for dissolution performance as described in example 6.1.

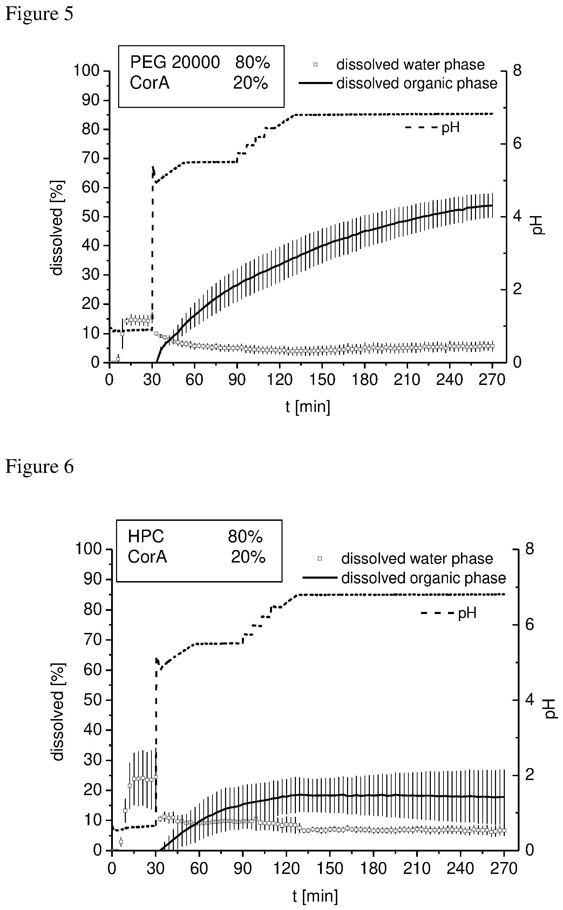

Figure 5 shows the pH profile and illustrates the biphasic dissolution profile of the amorphous formulation of corallopyronin A embedded in polyethylene glycol. As can be taken from figure 5, the formulation provided 53.9 % predicted absorption. The formulation thus provides good *in vitro* performance.

### Example 9: Biphasic dissolution test of the amorphous formulation of corallopyronin A embedded in hydroxypropyl cellulose

The amorphous formulation of corallopyronin A embedded in hydroxypropyl cellulose by film casting as prepared in example 2 was tested for dissolution performance as described in example 6.1.

Figure 6 shows the pH profile and illustrates the biphasic dissolution profile of the amorphous formulation of corallopyronin A embedded in hydroxypropyl cellulose. As can be taken from figure 6, the formulation provided 17.8 % predicted absorption.

### Comparative example 10: Pharmacological testing of pure corallopyronin A compound

### 10.1 Biphasic dissolution tests

Corallopyronin A was tested for dissolution performance as described in example 6.1. Figure 7 shows the pH profile and the biphasic dissolution profile of neat corallopyronin A compound (CorA). As can be taken from figure 7, the pure substance only provided 7.7 % predicted absorption.

### 10.2 Stability analysis

The stability of neat corallopyronin A compound was tested as described in example 6.3. The following table 4 summarises the results of the stability analysis.

**Table 4: Results of the stability analysis of Corallopyronin A**

| Storage Duration | Storage Condition | CorA Content [%] |
|---|---|---|
| 0 | | 99 |
| 7 days | 25 °C; 60% RH | 85 |
| 14 days | 25 °C; 60% RH | 76 |
| 28 days | 25 °C; 60% RH | 63 |
| 3 months | 25 °C; 60% RH | 39 |
| 7 days | 25 °C; 60% RH | 47 |
| 14 days | 40 °C; 75% RH | 31 |
| 28 days | 40°C; 75% RH | 17 |
| 3 months | 40 °C; 75% RH | 6 |

The results of table 4 show that the corallopyronin A content decreases fast at 25 °C and under conditions of 40 °C; after 7 days the corallpyronin A content at 25 °C falls below 90% and after 3 months below 40%; at accelerated conditions (40°C) the collopyronin A content falls below 50 % within 7 days and below 10 % within 3 months.

## Claims

1. A solid or liquisolid formulation comprising corallopyronin A, wherein the formulation comprises an amorphous solid dispersion of corallopyronin A embedded in a water-soluble polymer or corallopyronin A loaded onto porous silica.

2. The formulation according to claim 1, wherein the water-soluble polymer is selected from the group of polyvinylpyrrolidone, polyvinylpyrrolidone/vinylacetate copolymer, hydroxypropyl cellulose, hydroxypropyl methylcellulose and polyethylene glycol.

3. The formulation according to claim 1 or 2, wherein the water-soluble polymer comprises corallopyronin A in an amount in a range of ≥ 10 weight% to ≤ 50 weight%, preferably in an amount in a range of ≥ 15 weight% to ≤ 40 weight%, further preferred in an amount in a range of ≥ 20 weight% to ≤ 30 weight%, based on a weight of 100 weight% of the water-soluble polymer and Corallopyronin A.

4. The formulation according to any of the preceding claims, wherein the formulation comprises corallopyronin A embedded in polyvinylpyrrolidone.

5. The formulation according to claim 1, wherein corallopyronin A dispersed in a solvent, preferably in propylene carbonate, is adsorbed onto porous silica to form a liquisolid formulation.

6. The formulation according to any of the preceding claims, wherein the formulation is a solid or liquisolid oral formulation.

7. The formulation according to any of the preceding claims, wherein the formulation is formulated into a capsule, a tablet, granules, pills, pellets or micro-tablets.

8. A pharmaceutical solid dosage form, comprising the solid or liquisolid formulation according to claims 1 to 7.

9. The pharmaceutical solid dosage form according to claim 8, wherein the solid dosage form is selected from a capsule, a tablet, granules, pills, pellets or micro-tablets.

10. A process of manufacturing a solid or liquisolid formulation according to any of claims 1 to 7, comprising the steps of:
a) dispersing, dissolving or otherwise introducing corallopyronin A into a solvent to form a liquid mixture,
b) selecting a substrate from porous silica or a water-soluble polymer,
c) admixing the liquid mixture of step a) and the substrate of step b), and
d) optionally removing excess solvent from the mixture obtained in step c) to form a solid or liquisolid formulation.

11. The process of claim 10, wherein the solvent is an organic solvent selected from ethanol, methanol, isopropanol, dichloromethane, acetone, tert-butanol, propylene carbonate, a C6 to C12 triglyceride, preferably a C8 or C10 triglyceride, a polymer which is liquid at ambient temperature such as a polyethylene glycol, preferably selected from PEG 400, PEG 300 and PEG 200, or mixtures thereof.

12. The process according to claims 10 or 11, wherein the process is for manufacturing a solid formulation comprising corallopyronin A embedded in a water-soluble polymer, the process comprising the steps of:
a) dispersing, dissolving or otherwise introducing corallopyronin A in an organic solvent to form a liquid mixture,
b) selecting a water-soluble polymer,
c) admixing the liquid mixture of step a) and the water-soluble polymer of step b), and
d) removing excess solvent from the mixture obtained in step c) to form a solid formulation.

13. The process of claims 10 or 11, wherein the process is for manufacturing a solid or liquisolid formulation comprising corallopyronin A adsorbed to porous silica, the process comprising the steps of:
a) dispersing, dissolving or otherwise introducing corallopyronin A into a solvent to form a liquid mixture,
b) selecting a porous silica,
c) admixing the liquid mixture of step a) and the porous silica of step b), and
d) optionally removing excess solvent from the mixture obtained in step c) to form a solid or liquisolid formulation.

14. The process according to claims 10 to 13, wherein in a further step e) the solid or liquisolid formulation is formed into a capsule, a tablet, granules, pills, pellets or micro-tablets.

15. A solid or liquisolid formulation comprising corallopyronin A or a pharmaceutical solid dosage form obtained by the process according to claims 10 to 14.
